# FASCICULE DE BREVET EUROPEEN

(11) **EP 3 639 884 B1**
(45) Date de publication et mention de la délivrance du brevet: **16.12.2020**
(21) Numéro de dépôt: 19202358.8
(22) Date de dépôt: 10.10.2019
(51) Int. Cl.: A61N 1/05, A61N 1/372, A61N 1/375

(54) **SYSTÈME D'ACCOUPLEMENT ENTRE UNE CAPSULE CARDIAQUE AUTONOME ET SON OUTIL D'IMPLANTATION**
KUPPLUNGSSYSTEM ZWISCHEN EINEM LEADLESS HERZIMPLANTAT UND EINEM ZUGEHÖRIGEN IMPLANTATIONSWERKZEUG
COUPLING SYSTEM BETWEEN A LEADLESS CARDIAC IMPLANT AND ASSOCIATED IMPLANTATION TOOL

(30) Priorité: 17.10.2018 FR 1871199
(43) Date de publication de la demande: 22.04.2020
(73) Titulaire: Cairdac, 92160 Antony (FR)
(72) Inventeur: REGNIER, Willy, 91160 Longjumeau (FR); NGUYEN-DINH, An, 37520 La Riche (FR)
(74) Mandataire: Dupuis-Latour, Dominique

(56) Documents cités:
- WO-A1-2007/067231
- WO-A2-2011/057210
- US-A1- 2009 204 170
- US-A1- 2014 046 395
- US-A1- 2016 213 919
- US-A1- 2017 281 261
- US-A1- 2018 028 805
- US-B2- 8 340 780
- US-B2- 9 993 648

## Description

L'invention concerne les dispositifs médicaux implantables, notamment les dispositifs de type capsule autonome implantable.

L'invention concerne plus particulièrement ceux de ces dispositifs qui se présentent sous forme d'une capsule autonome implantée dans une cavité cardiaque (ventricule, oreillette ou même cavité cardiaque gauche artérielle), ci-après désignée "capsule autonome", "capsule *leadless*" ou simplement "capsule" (le caractère autonome de la capsule n'étant pas en soi une caractéristique nécessaire de l'invention). Ces capsules autonomes sont dépourvues de toute liaison physique à un dispositif principal implanté (tel qu'un boitier de générateur d'impulsions de stimulation) ou non implanté (périphérique externe tel que programmateur ou dispositif de monitoring pour le suivi à distance du patient), et sont dénommées pour cette raison "*leadless*", pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde.

L'invention n'est toutefois pas limitée à un type particulier de capsule, ni même d'implant *leadless*, et elle est applicable indifféremment à de nombreux autres types de dispositifs médicaux implantables, quelle que soit leur destination fonctionnelle, cardiaque ou autre, par exemple à des capsules destinées à diffuser *in situ* un agent pharmacologique actif. Dans ce cas d'une application cardiaque, la capsule surveille en continu le rythme du patient et délivre si nécessaire au cœur des impulsions électriques de stimulation, de resynchronisation et/ou de défibrillation en cas de troubles du rythme détecté par la capsule. La capsule peut être une capsule épicardique, fixée à la paroi extérieure du cœur, ou bien une capsule endocavitaire, fixée à la paroi intérieure d'une cavité ventriculaire ou auriculaire, ou bien encore une capsule fixée sur une paroi d'un vaisseau à proximité du myocarde.

Les US 2009/0171408 A1 (Solem), US 2017/0151429 A1 (Regnier) et WO 2018/122244 A1 (Regnier) décrivent divers exemples de telles capsules *leadless* intracardiaques.

Les capsules comprennent divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

L'invention concerne plus particulièrement les problèmes liés à l'implantation *in situ* de telles capsules lorsque celles-ci sont pourvues à leur extrémité distale d'un organe d'ancrage apte à pénétrer dans les tissus d'une paroi corporelle au site d'implantation choisi.

Un exemple typique d'un tel organe d'ancrage comprend une vis hélicoïdale saillante prolongeant axialement le corps de la capsule et destinée à pénétrer dans le tissu cardiaque par vissage au site d'implantation. Ce mode d'ancrage n'est toutefois pas limitatif de l'invention, qui peut s'appliquer également à d'autres types d'organes d'ancrage, par exemple mettant en œuvre des aiguilles, des crochets, des barbes, etc. pénétrant dans les tissus pour y assujettir de façon permanente le dispositif médical.

Dans le cas des capsules endocavitaires (c'est-à-dire des capsules à fixer à la paroi intérieure d'une cavité ventriculaire ou auriculaire, par opposition aux capsules épicardiques, fixées à la paroi extérieure du cœur), la "délivrance", c'est-à-dire à la mise en place au site d'implantation, consiste à monter la capsule à l'extrémité du cathéter-guide d'un outil d'implantation, puis à faire progresser l'ensemble le long du réseau veineux périphérique jusqu'au site choisi, par exemple l'apex de la cavité ventriculaire droite. Une fois le site d'implantation atteint, le praticien imprime à la capsule par l'intermédiaire du cathéter-guide des mouvements combinés i) de translation axiale, pour faire progresser la capsule de manière à la projeter vers l'avant jusqu'à toucher la paroi cardiaque et l'appuyer contre celle-ci puis ii) de rotation de la capsule autour de son axe, pour visser l'organe d'ancrage dans l'épaisseur de la paroi cardiaque. Une fois la capsule fermement ancrée dans la paroi cardiaque, l'opérateur procède au "largage" de la capsule, c'est-à-dire à sa désolidarisation d'avec l'outil d'implantation, la capsule devenant alors totalement autonome.

L'une des difficultés est, au stade de la fixation de la capsule dans la paroi, d'éviter tout risque de carottage des tissus du fait d'un vissage excessif. Pour cela, il est impératif, au moment du vissage de l'organe d'ancrage, de ne pas dépasser un couple limite (ci-après "couple de carottage") au-delà duquel la vis d'ancrage risquerait de déchirer localement les tissus sous l'effet de la rotation de la vis sans avance de celle-ci, jusqu'à provoquer une lacération des tissus et, à l'extrême, une perforation de la paroi avec risque de tamponnade (en particulier dans le cas d'une implantation dans une paroi mince telle que le septum interauriculaire ou la zone apicale du ventricule droit).

Le WO 2017/202724 A1 (Ollivier) propose une solution de couplage sécurisé combinant une capsule et un outil d'implantation spécifiques.

Ce document décrit des moyens de solidarisation en translation et en rotation de l'extrémité distale du cathéter-guide de l'outil d'implantation avec la région proximale de la capsule (celle opposée à l'organe d'ancrage). Ces moyens sont découplables pour pouvoir larguer la capsule une fois celle-ci en place, et ils incorporent un mécanisme débrayable simple permettant lors du vissage de l'organe d'ancrage de limiter le couple appliqué à la capsule par l'outil d'implantation.

Ce mécanisme est constitué par un ressort hélicoïdal utilisé en compression radiale (c'est-à-dire pour son effet de striction). Ce ressort est monté à l'extrémité distale du cathéter-guide et il est enroulé autour d'une tige d'arrimage de la capsule prolongeant celle-ci côté proximal. Le ressort joue alors un double rôle de moyen de liaison (débrayable) et de limiteur de couple à l'encontre d'une action de vissage excessive qui pourrait entrainer un carottage des tissus. En effet, en fin de vissage, lorsque le corps de la capsule vient en appui contre la paroi, le couple supplémentaire venant de la réaction de la vis est absorbé par la liaison entre le ressort et la tige d'arrimage de la capsule : le praticien peut alors poursuivre sans risque la rotation du cathéter-guide au-delà de ce qui est strictement nécessaire pour un vissage complet de la capsule dans la paroi.

Un inconvénient de cette technique connue est la nécessité de prévoir en partie proximale de la capsule (c'est-à-dire du côté opposé à la vis d'ancrage), une tige d'arrimage suffisamment longue pour qu'elle puisse être emprisonnée par le ressort de l'outil d'implantation. Cette exigence augmente nécessairement la longueur hors-tout de la capsule, ce qui va à l'encontre des exigences de miniaturisation extrême imposées aux capsules *leadless.* Une longueur accrue rend par ailleurs la capsule plus difficile à faire progresser le long du réseau veineux périphérique jusqu'au site choisi.

Un autre inconvénient de cette technique connue est la difficulté qu'il peut y avoir si, après implantation et largage de la capsule, le praticien constate au vu des tests électriques que le site d'implantation n'est pas optimal et souhaite explanter la capsule pour la réimplanter en un site voisin, ou encore s'il souhaite explanter définitivement une capsule déjà en place. Il devra alors manœuvrer l'outil d'implantation (qui avait été découplé de la capsule) de manière à venir replacer le ressort hélicoïdal contre la tige d'arrimage de la capsule puis tenter, par des mouvements combinés de poussée et de rotation du cathéter, de replacer ce ressort autour de la tige pour pouvoir exercer à nouveau sur cette dernière l'effort de serrage radial nécessaire pour coupler le cathéter à la capsule. Ces manœuvres sont extrêmement délicates, voire irréalisables dans des conditions opératoires réelles, ce qui constitue une limitation sérieuse aux possibilités d'utilisation de ce système connu.

D'autres solutions de couplage d'une capsule *leadless* à un outil d'implantation spécifique ont été proposées, mais aucune ne procure une double fonctionnalité i) de limitation intrinsèque du couple de rotation exercé sur la capsule par l'outil au moment de la pose et ii) de réversibilité pour permettre une explantation aisée, *a fortiori* grâce à une structure mécanique simple, fiable, et facile et peu coûteuse à industrialiser.

Ainsi, les US 2009/204170 A1 et WO 2007/067231 A1 décrivent une capsule implantable munie à son extrémité arrière (proximale) d'une tige de rétention ("retainer pin" ou "retainer shaft") orientée transversalement (c'est-à-dire radialement) par rapport au corps de la capsule. L'outil d'implantation comporte à son extrémité frontale (distale) deux doigts saillants approximativement plans et parallèles, orientés axialement vers l'arrière de la capsule et emprisonnant entre eux la tige de rétention transversale de cette dernière. L'un des doigts porte une empreinte creuse arrondie recevant la tige introduite entre les deux doigts. Pour bloquer la tige en position dans l'espace formé entre les deux doigts, un fil est intercalé en force entre la face libre de la tige et le doigt situé en vis-à-vis. Pour libérer la capsule une fois celle-ci mise en place, le fil ("locking pull-wire") est retiré, ce qui relâche la pression sur la tige et libère celle-ci de l'espace où elle se trouvait entre les doigts, ce qui a pour conséquence de découpler la capsule d'avec l'outil d'implantation. Dans une variante décrite par le WO 2007/067231 A1, la tige est pincée entre les doigts du seul fait de l'élasticité propre de ces derniers. La libération de la capsule se fait alors en enfonçant un "push-wire" qui expulse la tige hors de l'espace entre les doigts. Le mécanisme décrit par ces deux documents ne procure, par nature, aucune limitation intrinsèque du couple de rotation exercé sur la capsule par l'outil. Il n'est pas non plus directement ni simplement réversible.

Le US 2016/213919 A1 décrit une capsule avec une extrémité arrière en forme de champignon ou de crochet, destinée à faciliter une explantation au moyen d'un "lasso" venant accrocher cette extrémité arrière afin de pouvoir ensuite depuis l'extérieur guider et approcher un cathéter jusqu'à la capsule. Cette forme particulière ne procure ni limitation de couple ni réversibilité au sens précisé plus haut.

Le US 2018/028805 A1 décrit un outil d'implantation doté d'un organe de couplage en forme de griffes flexibles venant saisir et emprisonner l'extrémité arrière, en forme de champignon, de la capsule. Ce système assure une fonction intrinsèque de limitation du couple de rotation, dans la mesure où les doigts vont tourner dans le vide lorsque la capsule opposera une certaine résistance à la rotation. Pour détacher la capsule des griffes de l'outil, il est nécessaire de prévoir un mécanisme additionnel car le découplage capsule/outil n'est pas automatique ; une tige du cathéter doit être introduite dans la lumière centrale et manœuvrée pour repousser axialement l'extrémité arrière de la capsule et la libérer des griffes par une déformation élastique de ces dernières. Outre sa complexité structurelle, ce mécanisme ne procure aucune réversibilité, l'explantation nécessitant de mettre en œuvre une technique telle que celle décrite par le US 2016/213919 A1 précité.

Enfin, le US 9 993 648 B2 décrit une structure d'outil d'implantation avec un tube muni à son extrémité distale d'une forme en entonnoir, approximativement conique, dans laquelle vient se loger l'extrémité arrière de la capsule, en forme de champignon. Deux fentes réalisées dans l'entonnoir donnent à celui-ci une certaine élasticité radiale qui lui permet de pincer l'extrémité arrière de la capsule lorsque celle-ci est insérée à force dans le fond de l'entonnoir. Ce mode de couplage outil/capsule est théoriquement réversible, mais une telle manœuvre serait en pratique très difficile à envisager du fait de l'absence de préguidage et de précentrage mutuels de la capsule et de la tête de l'outil pour que les deux formes puissent s'emboîter. En particulier, la face frontale plane de la tête de l'outil est susceptible de créer une situation de blocage si l'axe de la capsule et l'axe de l'outil ne sont pas alignés au moment du rapprochement de ces deux éléments. Or, un alignement même approximatif de ces deux axes est en pratique inenvisageable dans les conditions réelles d'une intervention chirurgicale, où l'extrémité arrière, libre, de la capsule oscille en permanence au gré des battements cardiaques et des turbulences du flux sanguin environnant.

L'un des buts de l'invention est de proposer un système d'accouplement d'une capsule autonome à son outil d'implantation qui soit aisé à mettre en œuvre et de structure simple, tout en intégrant un mécanisme limiteur de couple procurant lors de l'implantation une sécurité absolue à l'encontre de tout risque de carottage et de lacération des tissus du fait d'un vissage excessif de la capsule dans la paroi.

Un autre but de l'invention est de proposer un tel système d'accouplement qui soit parfaitement réversible, c'est-à-dire avec lequel après un premier découplage l'outil d'implantation puisse être à nouveau couplé à la capsule de façon simple, sûre et rapide, de manière à permettre un dévissage de cette capsule en vue d'une réimplantation à un autre site (ou d'une explantation définitive).

Un autre but encore de l'invention est de proposer une telle capsule dont le coût de fabrication soit réduit, notamment grâce à des pièces en nombre réduit et de formes simples, et par l'utilisation de technologies et de composants éprouvés dans des applications similaires.

À cet effet l'invention propose un ensemble comprenant les éléments énoncés dans le préambule de la revendication 1, connus notamment d'après le WO 2017/202724 A1 précité, avec les éléments originaux énoncés dans le préambule de cette revendication 1.

Les sous-revendications visent des modes de réalisation particuliers de l'invention, subsidiaires et avantageux.

On va maintenant décrire un exemple de réalisation de la présente invention en référence aux dessins annexés où les mêmes références désignent d'une figure à l'autre des éléments identiques ou fonctionnellement semblables.
La Figure 1 est une vue d'ensemble illustrant l'outil et la capsule de l'invention, en situation pendant une opération d'implantation dans le ventricule droit d'un myocarde.
La Figure 2 illustre la capsule de l'invention couplée au cathéter à l'intérieur du manchon de protection, avant déploiement et vissage de la capsule.
La Figure 3 est une vue agrandie en perspective de l'extrémité du cathéter avec sa pièce d'accostage et le fil de sécurité, à l'intérieur du manchon de protection.
La Figure 4 est une vue en perspective montrant, isolément, la partie arrière (proximale) de la capsule, avec la forme conjuguée destinée au couplage avec la pièce d'accostage du cathéter.
La Figure 5 illustre en perspective la pièce d'accostage du cathéter et la forme conjuguée de la capsule, couplées ensemble pour permettre l'entrainement en rotation et en translation de la capsule par l'outil d'implantation.
La Figure 6 est une coupe par un plan axial, selon VI-VI de la Figure 7, de la pièce d'accostage du cathéter et de la partie arrière de la capsule, couplées ensemble.
La Figure 7 est une coupe par un plan axial, selon VII-VII de la Figure 6, de la pièce d'accostage du cathéter et de la partie arrière de la capsule, couplées ensemble.
La Figure 8 illustre en perspective, isolément, une variante de la pièce d'accostage des figures précédentes.
La Figure 9 illustre en perspective la pièce d'accostage de la Figure 8 et la partie arrière de la capsule, au moment de l'accostage entre ces deux éléments juste avant leur couplage.
La Figure 10 illustre en perspective la pièce d'accostage et la partie arrière de la capsule illustrées Figure 9, couplées ensemble.
La Figure 11 illustre en perspective la capsule couplée à la pièce d'accostage du cathéter, l'ensemble étant déployé hors du manchon tubulaire de protection dans une situation correspondant typiquement à une phase de recapture de la capsule en vue d'une explantation de celle-ci.
La Figure 12 est une coupe par un plan axial de la pièce d'accostage et de l'extrémité distale du manchon de protection dans la configuration de la Figure 11, illustrant notamment le rôle joué au cours de cette opération par le cône d'insertion formé à l'arrière de la pièce d'accostage.

On va maintenant décrire un exemple de réalisation d'un ensemble selon l'invention.

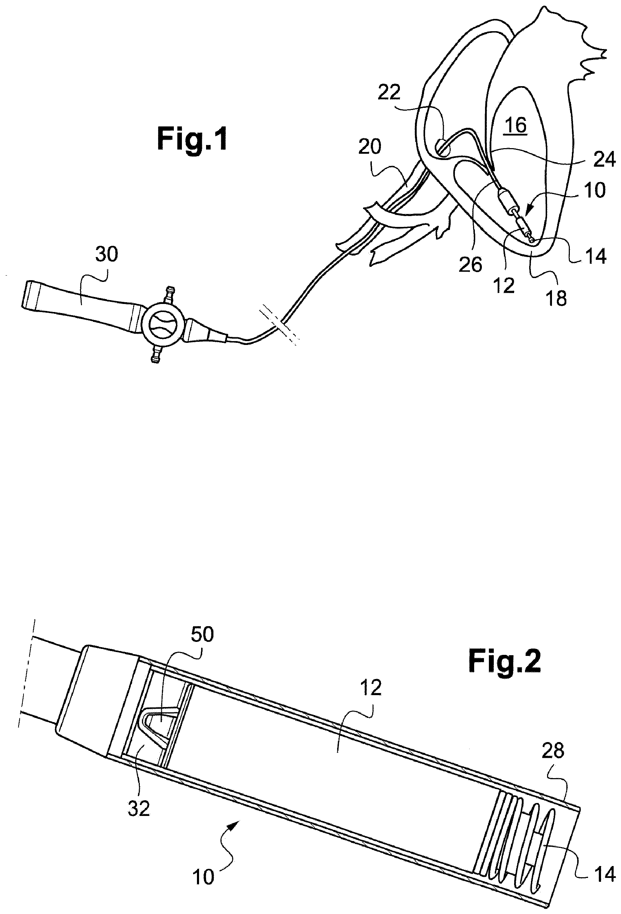

La Figure 1 illustre un outil d'implantation et une capsule autonome *leadless* selon l'invention, en situation pendant une opération de mise en place de cette capsule dans le ventricule droit d'un myocarde.

La capsule, référencée 10 (et représentée plus en détail sur la Figure 2), comprend de façon en elle-même connue un corps tubulaire 12 pourvu à l'une de ses extrémités d'une vis d'ancrage hélicoïdale saillante 14 prolongeant axialement le corps tubulaire 12 et solidaire de celui-ci en rotation. La vis d'ancrage comprend dans sa partie distale une longueur de l'ordre de 1,5 à 2 mm de spires non jointives, destinées à pénétrer dans le tissu cardiaque de façon à y assujettir la capsule.

Dans l'exemple illustré, la capsule est implantée dans le ventricule droit 16 d'un cœur, au fond de ce ventricule dans la région de l'apex 18. L'accès au ventricule droit 16 se fait par la veine cave 20 via le sinus 22 puis la valve tricuspide 24.

L'outil d'implantation comprend à cet effet un cathéter 26 avec, à son extrémité distale, un manchon tubulaire de protection 28 logeant la capsule, celle-ci étant progressivement déployée hors du manchon jusqu'à accostage avec la paroi cardiaque. À l'extrémité opposée, proximale, le cathéter 26 est relié à une poignée de manœuvre 30 manipulée par le praticien.

Ici et dans la suite, le terme "proximal" (ou "arrière") sera considéré par rapport à l'outil d'implantation, c'est-à-dire en direction de la poignée manipulée par le praticien ; de même, le terme "distal" (ou "avant") se réfèrera à une direction opposée, donc proche du site d'implantation et de la vis d'ancrage 14 de la capsule. Ces directions proximale et distale correspondent respectivement à la gauche et à la droite sur les différentes figures annexées. De même, le terme "axial" sera utilisé en référence à l'axe de la capsule, c'est-à-dire la plus grande dimension de la capsule, en l'espèce l'axe *D* du corps cylindrique 12, une direction "radiale" étant une direction située dans un plan perpendiculaire à la direction axiale.

À l'aide de diverses manettes et boutons le praticien oriente le cathéter progressivement le long de la veine cave 20 puis oriente l'extrémité distale de façon précise jusqu'à l'accostage au fond du ventricule 16. Il imprime alors au cathéter 26 depuis la poignée un double mouvement de translation pour appuyer l'extrémité distale de la capsule contre la paroi, et de rotation pour visser la capsule de manière à l'ancrer dans la paroi.

La Figure 2 montre sous forme plus agrandie la capsule 10 avec son corps tubulaire 12 logé à l'intérieur du manchon de protection 28 dans une configuration dite "position rétractée", où le manchon 28 abrite la capsule, et notamment la vis d'ancrage 14 pendant la progression dans le réseau veineux, au passage de la valve, etc. Le manchon 28 protège également les tissus environnants des risques de déchirements potentiels par la vis avant que celle-ci n'atteigne sa position définitive.

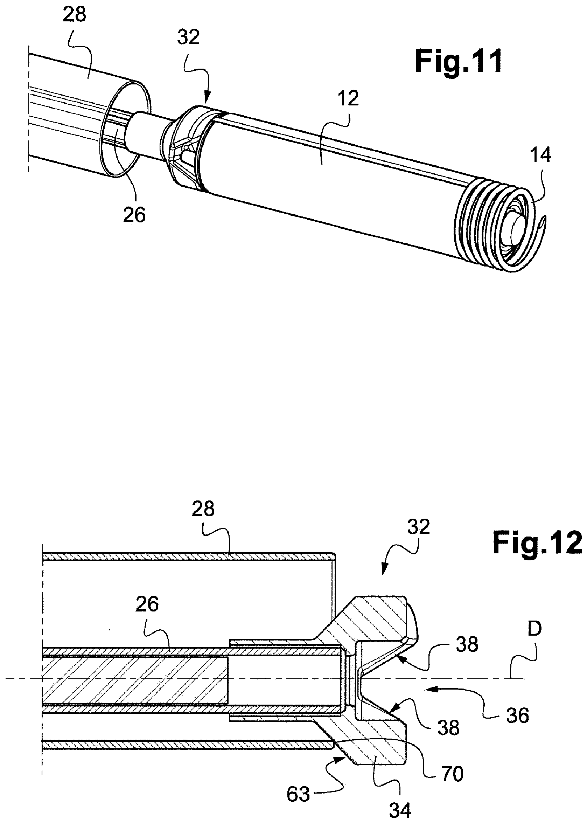

Une fois que la capsule a touché le site d'implantation, le praticien opère une manœuvre de translation de la capsule en direction distale, ce qui a pour effet de déployer celle-ci hors du manchon tubulaire de protection 28 (dans une configuration telle que celle illustrée Figure 11). Par un mouvement de rotation axiale il visse alors la capsule dans la paroi cardiaque jusqu'à ce que la face avant du corps tubulaire 12, qui porte une électrode (non représentée) vienne en appui contre cette paroi. Dans cette position, qui est la position définitive de la capsule, le praticien peut alors découpler le cathéter d'avec la capsule, puis procéder au retrait du cathéter hors de l'organisme par une manœuvre inverse de celle qui avait permis l'implantation.

L'invention concerne plus précisément la manière de coupler la capsule 10 au cathéter 26, par un moyen permettant d'assurer la transmission du couple de rotation depuis l'extrémité proximale du cathéter au niveau de la poignée de manœuvre 30, jusqu'à l'extrémité distale de la capsule 10 portant la vis d'ancrage 14.

De façon caractéristique, ce moyen met en œuvre un système de transmission et de limitation du couple appliqué à la capsule par le cathéter (et donc du couple exercé par la vis d'ancrage 14 sur les tissus cardiaques) qui à la fois : i) garantit un vissage complet de la capsule dans le tissu cardiaque au site d'implantation ; ii) évite tout carottage de la paroi cardiaque ; et iii) permet au praticien en cas de difficulté de récupérer la capsule après que celle-ci a été larguée, notamment par réversibilité du couplage.

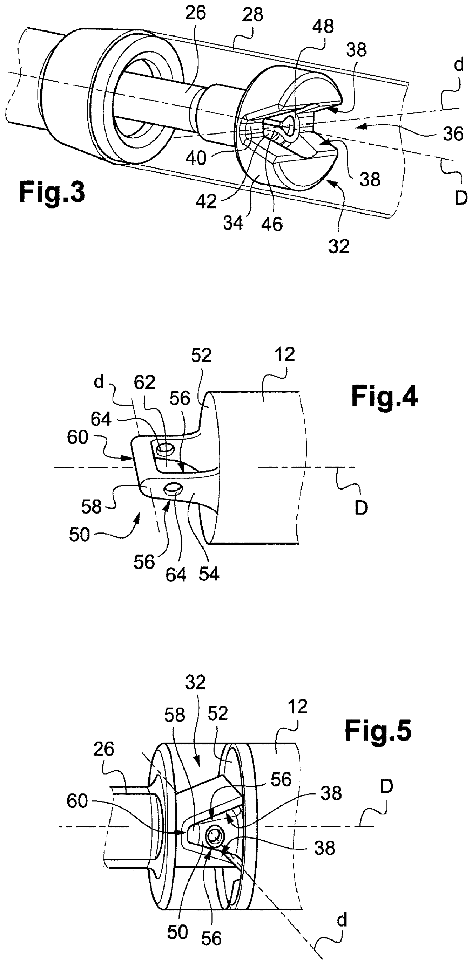

Les Figures 3 à 7 illustrent un mode de réalisation de ce moyen de couplage selon l'invention.

L'extrémité distale du cathéter 26 est munie d'un organe de couplage 32, ci-après désigné "pièce d'accostage", coopérant avec un organe de couplage conjugué 50 en partie proximale du corps de la capsule 10, partie ci-après désignée "arrière de la capsule".

La pièce d'accostage 32 comprend un corps cylindrique 34 dont le diamètre lui permet de pénétrer et coulisser dans le manchon tubulaire de protection 28. Ce corps cylindrique est réalisé avantageusement en une matière plastique telle qu'un polymère de type PET (poly(téréphtalate d'éthylène)) ou PEEK (polyétheréthercétone) ou autre matière plastique injectable, éventuellement avec revêtement d'un film de parylène (polymère de poly(p-xylylène), matière connue pour ses propriétés hydrophobes et de faible coefficient de friction. En variante, il peut aussi être réalisé en un matériau métallique tel qu'un acier inoxydable (par exemple 316L), le titane ou le tantale, avec ou sans revêtement pour améliorer le coefficient de friction.

Côté distal, le corps cylindrique 34 comporte une empreinte constituée d'un évidement 36 symétrique en forme approximative de V, avec deux faces inclinées 38 s'étendant selon des plans situés de part et d'autre de l'axe central *D*, avec une configuration de dièdre symétrique, l'axe *d* de ce dièdre s'étendant dans une direction radiale. L'inclinaison des faces 38 par rapport à l'axe *D* est choisie de manière que ces faces forment entre elles un angle compris entre 10° et 80°, de préférence 30° à 60°, plus précisément 45° dans l'exemple illustré sur les Figures 3 à 7.

Le fond 40 de l'évidement 36 présente à l'endroit où se raccordent les faces inclinées 38 une forme arrondie, cylindrique de révolution dans l'exemple illustré.

Le corps cylindrique 34 est traversé axialement de part en part par un orifice axial 42 débouchant dans une lumière interne 44 du cathéter 26. Cet orifice 42 permet le passage d'un fil de sécurité 46 traversant le cathéter 26 sur toute sa longueur jusqu'à la poignée de manœuvre à l'extrémité proximale ; le fil de sécurité 46 forme à l'extrémité distale une boucle 48 s'étendant approximativement dans l'espace défini entre les faces inclinées 38 de l'évidement 36.

L'évidement 36 de la pièce d'accostage 32 est tourné vers l'arrière 50 de la capsule, qui est constitué en un organe de couplage conjugué susceptible de coopérer avec l'évidement 36.

Plus précisément, le corps cylindrique 12 de la capsule est fermé à l'arrière par un capot 52 portant une excroissance 54 dont la forme et les dimensions lui permettent de pénétrer à l'intérieur de l'évidement 36 situé en vis-à-vis. Le matériau de cette excroissance 54 peut être le même que celui du capot de fermeture 52 et du corps tubulaire 12, à savoir un matériau métallique tel que le titane, un acier inoxydable (par exemple 316L), le tantale, ou un alliage nickel-titane de type nitinol, éventuellement avec un revêtement pour améliorer le coefficient de friction. En variante, il peut aussi être réalisé en un matériau tel qu'un polymère de type PET ou PEEK.

L'excroissance 54 comprend deux surfaces 56 symétriques par rapport à l'axe *D* et formant ensemble une surface réglée avec deux plats inclinés 56 allant en se rétrécissant vers l'arrière. Les deux plats 56 forment ainsi approximativement un V, dont l'angle d'ouverture est inférieur à celui du V de la pièce d'accostage 32 défini entre les deux faces inclinées 38. À son extrémité proximale 58, l'excroissance 54 présente une surface arrondie 60, par exemple une surface cylindrique de révolution d'axe *d* s'étendant dans un plan radial perpendiculairement à l'axe principal *D*. Le rayon de courbure de la surface arrondie 60 est inférieur à celui de la surface cylindrique 40 de la pièce d'accostage 32 en vis-à-vis, de telle sorte que l'excroissance 54 puisse pénétrer à l'intérieur de l'évidement 36 de la pièce d'accostage jusqu'au fond de celle-ci.

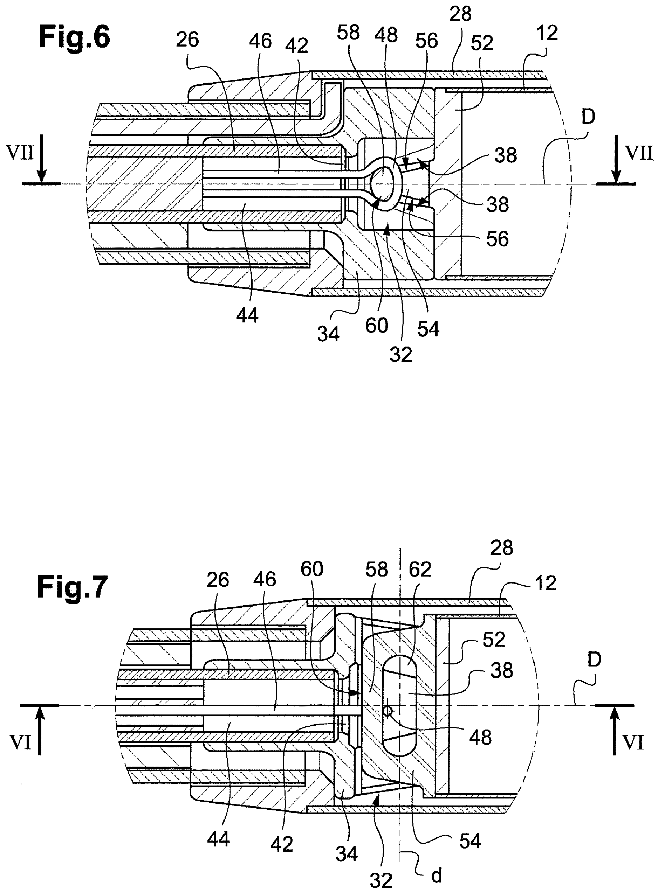

Enfin, l'excroissance 54 comporte un évidement 62 permettant le passage de la boucle 48 du fil de sécurité 46 (comme cela est illustré notamment sur les Figures 6 et 7) et donc l'assujettissement du corps de la capsule à ce fil de sécurité. En variante, l'excroissance 54 peut comporter des orifices tels que 64 (voir Figure 4) formés dans des directions parallèles à l'axe transversal *d* et permettant l'enfilage du fil de sécurité.

Une première fonction de l'ensemble constitué de la pièce d'accostage 32 et de l'organe de couplage conjugué 50 à l'arrière de la capsule est d'assurer un préguidage entre le corps de la capsule 12, au niveau de son extrémité proximale, avec l'extrémité du cathéter 26 au moment où la capsule est rapprochée de son outil d'implantation par mise en tension du fil de sécurité 46 : ce préguidage est assuré par la forme en V de l'évidement 36 et la forme saillante de l'excroissance 54 en vis-à-vis, avec réduction progressive du jeu de guidage entre la pièce d'accostage 32 et l'extrémité arrière de la capsule au fur et à mesure de leur rapprochement mutuel à l'insertion dans le manchon tubulaire de protection 28.

Une seconde fonction de l'ensemble constitué de la pièce d'accostage 32 et de l'organe de couplage conjugué 50 est d'assurer une limitation du couple de rotation transmis à la capsule par le cathéter via le manchon tubulaire 28.

Tout d'abord, une fois que l'arrière de la capsule et le manchon tubulaire ont été couplés ensemble par rapprochement mutuel et mise sous tension du fil de sécurité 46, l'excroissance 54 est plaquée au fond de l'évidement 36 de sorte qu'une translation appliquée au cathéter 26 et transmise à la capsule par le manchon tubulaire 28 assure le déploiement de la capsule hors du manchon tubulaire de protection 28 et la venue en contact avec la paroi cardiaque.

Le praticien peut alors commencer à visser la capsule dans la paroi cardiaque en imprimant un mouvement de rotation axiale au cathéter 26. Du fait du contact entre, d'une part, les faces inclinées 38 de la pièce d'accostage 32 et, d'autre part, la surface en vis-à-vis 56 de l'excroissance 54, le couple de rotation est transmis à la capsule.

En fin de vissage, la face avant de la capsule qui touche la surface de la paroi cardiaque exerce sur la vis d'ancrage une force de réaction axiale qui serait susceptible de produire un carottage. Mais comme le contact précité entre les faces inclinées 38 et la surface en vis-à-vis 56 est un contact frottant (frottement de la surface cylindrique 60 sur le plan incliné des faces 38), du fait du couple de réaction qui augmente de façon relativement brusque la limite du frottement est rapidement atteinte et la pièce d'accostage 32 sort de l'excroissance 54, d'une manière comparable à celle d'une lame de tournevis qui s'échappe de l'empreinte d'une tête de vis lorsque la vis se bloque. On réalise ainsi la fonction recherchée de limitation du couple et de débrayage entre la capsule et la pièce d'accostage du cathéter.

Le couple de rotation à partir duquel ce phénomène se produit peut être ajusté par un choix particulier : i) des matériaux respectifs de la pièce d'accostage 32 et de l'arrière 50 de la capsule (un choix de matériaux qui donne un contact frottant métal-sur-polymère dans l'exemple décrit) ; ii) de l'angle d'ouverture de l'empreinte formée par l'évidement 36, essentiellement l'angle des faces inclinées 38 de V ; et iii) de la forme de l'excroissance 54, et notamment de son extrémité 58 (forme cylindrique 60 dans l'exemple illustré).

On notera que, pour ce qui concerne le contact entre, respectivement, la pièce d'accostage 32 et l'arrière 50 de la capsule, l'invention n'est pas limitée à un contact frottant métal/polymère (comme dans l'exemple illustré), mais peut s'appliquer aussi bien à un contact frottant de type polymère/métal, métal/métal ou polymère/polymère, avec les matériaux qui ont été indiqués plus haut à titre d'exemple.

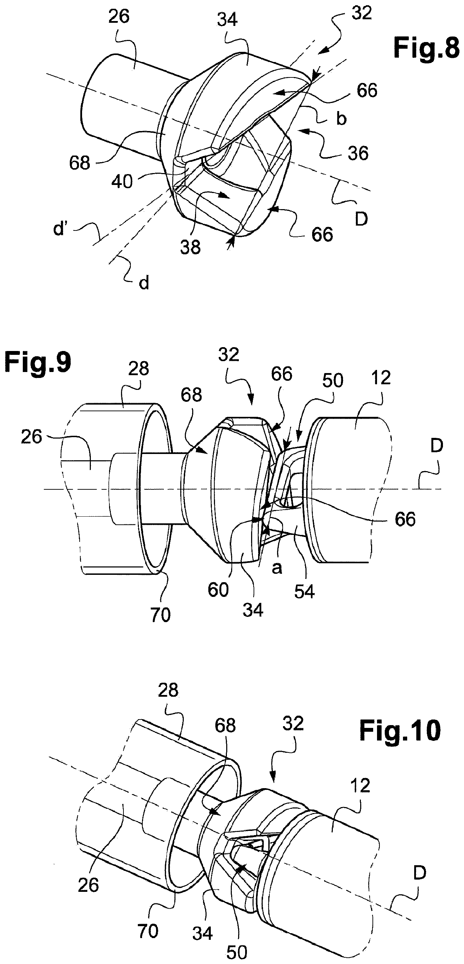

Le couple limite à partir duquel la pièce d'accostage 32 et l'arrière 50 de la capsule se déboitent est choisi inférieur à la limite de carottage (limite au-delà de laquelle la vis d'ancrage risquerait de déchirer localement les tissus sous l'effet de la rotation de la vis sans avance de celle-ci) tout en étant suffisant pour permettre la pénétration de la vis d'ancrage jusqu'à vissage complet (tissu en contact avec la face avant de la capsule). Une valeur appropriée du couple limite est typiquement inférieure à 5 N.cm. Les Figures 8 à 12 illustrent une variante perfectionnée de la pièce d'accostage 32.

Dans cette variante, la pièce d'accostage 32 comprend une surface frontale tournée vers la capsule constituée de deux demi-faces frontales 66 qui viennent prolonger en direction radiale chaque face inclinée respective 38 de l'évidement 36. À la différence du mode de réalisation des Figures 3 à 7 où ces demi-faces étaient coplanaires et s'étendaient dans un plan radial, dans cette variante des Figures 8 à 12 les demi-faces frontales 66 sont inclinées dans des sens opposés par rapport à un plan radial (plan contenant un axe radial *d* suivant lequel s'étend le fond 40 de l'évidement 36). L'angle d'inclinaison des demi-faces frontales 66 par rapport à un plan radial, c'est-à-dire l'angle entre les directions *d*'et *d* sur la Figure 8) est compris entre 5 et 15°, dans un sens pour l'une des demi-faces frontales et dans le sens opposé pour l'autre demi-face frontale.

Du fait de l'inclinaison des demi-faces frontales 66, lors de l'accostage de la capsule contre la pièce d'accostage 32 il n'y a plus de position stable et la capsule est automatiquement dirigée vers le fond de l'empreinte de l'évidement 36.

Cette caractéristique est particulièrement avantageuse lorsque, après avoir implanté la capsule et découplé celle-ci d'avec l'outil, puis après un premier test électrique défavorable, le praticien souhaite accoupler à nouveau l'outil à la capsule, typiquement pour la dévisser et la revisser à un site d'implantation voisin, estimé meilleur.

Tout d'abord, du fait du caractère réversible du mécanisme que l'on vient de décrire, un couple de rotation exercé en sens inverse sur la pièce d'accostage 32 sera transmis au corps tubulaire 12 et donc à la vis hélicoïdale 14, ce qui permet de dévisser la capsule et donc de la détacher progressivement de la paroi cardiaque.

Il sera alors aisé de rapprocher la pièce d'accostage 32 de l'arrière 50 de la capsule par une traction sur le fil de sécurité. Au moment où l'arrière 50 de la capsule vient en contact avec la pièce d'accostage 32, la configuration est celle de la Figure 9, avec la surface d'extrémité 60 de l'excroissance 54 qui vient en appui contre les demi-faces frontales 66. Pour éviter tout risque de blocage, et pour être certain que l'arrière 50 de la capsule sera toujours en contact avec au moins l'une des demi-faces inclinées 66, la longueur en direction axiale a (Figure 9) de l'excroissance 54 est choisie supérieure à la plus grande dimension *b* (Figure 8) entre les sommets de raccordement situés aux extrémités supérieures, diamétralement opposées, des demi-faces frontales respectives. En continuant de tirer sur le fil de sécurité, la surface d'extrémité 60 glisse le long des demi-faces frontales inclinées 66 jusqu'à pénétrer dans l'évidement 36, réalisant à nouveau le couplage de la capsule à la pièce d'accostage 32, avec une configuration telle que celle illustrée Figures 10 et 11.

On notera que ces manœuvres se font avec la pièce d'accostage et la capsule entièrement sorties du manchon de protection 28. De fait, le diamètre du cathéter 26 étant inférieur au diamètre intérieur du manchon tubulaire de protection 28, lors de la manœuvre il peut se produire un désalignement des axes, comme illustré Figure 12. Pour faciliter l'insertion de la pièce d'accostage 32 et la réintroduction de la capsule à l'intérieur du manchon tubulaire de protection 28, la pièce d'accostage 32 est avantageusement raccordée à l'extrémité distale du cathéter 26 par une partie 68 de forme approximativement conique, qui vient alors buter contre le rebord 70 de l'extrémité distale du cathéter 28. Le cône d'insertion de la partie de raccord 68 permet de réduire progressivement le désalignement, jusqu'à réalignement complet de la pièce d'accostage 32 au moment où celle-ci commence à entrer dans le manchon tubulaire de protection 28.

## Revendications

1. Un ensemble comprenant une capsule autonome implantable (10) et un outil (26, 30) pour le guidage et l'ancrage de la capsule à un site d'implantation (18), dans lequel :
la capsule comprend un corps tubulaire (12) pourvu à son extrémité distale d'un organe d'ancrage (14) apte à pénétrer dans un tissu d'une paroi d'un organe, et à son extrémité proximale d'un organe de couplage (50) apte à coopérer avec un organe de couplage conjugué (32) de l'outil ;
l'outil comprend un cathéter (26) pourvu à son extrémité distale dudit organe de couplage conjugué (32) ;
l'organe de couplage (50) de la capsule et l'organe de couplage conjugué (32) de l'outil présentent des formes respectives complémentaires (54, 56, 58, 60 ; 36, 38, 40) aptes à permettre la transmission d'un couple d'entrainement en rotation de la capsule par le cathéter ;
l'ensemble comprend en outre un moyen de jonction (46, 48, 62) apte à produire sélectivement en direction axiale un effort de rapprochement mutuel et d'appui de l'organe de couplage (50) de la capsule contre l'organe de couplage conjugué (32) de l'outil, propre à solidariser en rotation et en translation la capsule (10) avec le cathéter (26) puis découpler la capsule du cathéter par un relâchement dudit effort,
cet ensemble étant **caractérisé en ce que** :
la forme de l'organe de couplage conjugué (32) de l'outil comprend côté distal, en section droite par un plan axial, un évidement (36) tourné vers l'arrière de la capsule, avec une forme en V (36) comprenant deux faces inclinées, et
la forme de l'organe de couplage (50) de la capsule comprend à son extrémité proximale une surface convexe (60) apte à venir en appui frottant et glissant contre les faces inclinées divergentes (38) de la forme en V (36).

2. L'ensemble de la revendication 1, dans lequel la forme en V (36) est une forme de dièdre avec les deux faces inclinées divergentes (38) s'étendant dans des plans respectifs.

3. L'ensemble de la revendication 2, dans lequel la droite d'intersection (d) du dièdre s'étend radialement.

4. L'ensemble de la revendication 2, dans lequel l'angle du dièdre est compris entre 10° et 80°, de préférence compris entre 30° et 60°.

5. L'ensemble de la revendication 1, dans lequel l'organe de couplage conjugué (32) de l'outil comprend côté distal une surface frontale apte à venir en appui contre l'extrémité proximale de la capsule et comprenant deux demi-faces frontales (66) prolongeant radialement chaque face inclinée (38) respective de la forme en V (36).

6. L'ensemble de la revendication 5, dans lequel les deux demi-faces frontales (66) s'étendent dans des plans respectifs inclinés dans des sens opposés par rapport à un plan radial.

7. L'ensemble de la revendication 6, dans lequel l'angle d'inclinaison des plans des demi-faces frontales (66) respectives par rapport au plan radial est compris entre 5° et 15°.

8. L'ensemble de la revendication 1, dans lequel l'organe de couplage conjugué (32) de l'outil est relié côté proximal au cathéter par une partie de raccord évasée dont la dimension en section droite radiale augmente progressivement à partir du cathéter en allant vers l'organe de couplage conjugué.

9. L'ensemble de la revendication 1, dans lequel la surface convexe (60) de l'organe de couplage de la capsule est une surface cylindrique dont l'axe (d) est orienté radialement.

10. L'ensemble de la revendication 1, dans lequel le matériau de l'organe de couplage (50) de la capsule est un matériau métallique et le matériau de l'organe de couplage conjugué (36) de l'outil est une matière plastique polymère.

11. L'ensemble de la revendication 1, dans lequel :
le matériau de l'organe de couplage de la capsule,
le matériau de l'organe de couplage conjugué de l'outil,
la forme de l'organe de couplage de la capsule, et
la forme de l'organe de couplage conjugué de l'outil sont choisis conjointement pour, à l'interface de contact entre la forme de l'organe de couplage (50) de la capsule et la forme de l'organe de couplage conjugué (36) de l'outil, produire une force de frottement de contact suffisante pour :
coupler la capsule (10) au cathéter (26) et autoriser l'entrainement en rotation de la capsule par le cathéter tant qu'un couple de réaction exercé par l'organe d'ancrage de la capsule est inférieur à un couple seuil prédéterminé, et
découpler la capsule (10) du cathéter (26) dès que le couple de réaction dépasse le couple seuil prédéterminé, et interrompre ainsi la transmission à la capsule du couple d'entrainement en rotation.

12. L'ensemble de la revendication 11, dans lequel le couple seuil prédéterminé est inférieur à 5 N.cm.

13. L'ensemble de la revendication 1, dans lequel :
le moyen de jonction comprend un fil de retenue (46, 48) logé dans une lumière (44) du cathéter (26) et débouchant (44) à l'extrémité distale du cathéter, et
le fil de retenue (46, 48) est assujetti à l'organe de couplage (50) de la capsule, de manière qu'une traction axiale exercée en direction proximale sur le fil de retenue sollicite l'organe de couplage (50) de la capsule en rapprochement contre l'organe de couplage conjugué (36) de l'outil.

14. L'ensemble de la revendication 1, dans lequel l'outil comprend en outre un manchon tubulaire de protection (30) porté par l'extrémité distale du cathéter (26) et définissant un volume intérieur apte à loger, avec un degré de liberté en coulissement axial : au moins la région proximale (52, 54) du corps tubulaire (12) de la capsule incluant l'organe de couplage (50) de la capsule ; l'organe de couplage conjugué (36) de l'outil ; et le moyen de jonction (46, 48, 62).

## Patentansprüche

1. Anordnung, umfassend eine implantierbare autonome Kapsel (10) und ein Werkzeug (26, 30) zur Führung und zur Verankerung der Kapsel an einer Implantationsstelle (18), wobei:
die Kapsel einen rohrförmigen Körper (12) umfasst, der an seinem distalen Ende mit einem Verankerungsorgan (14) versehen ist, das imstande ist, in ein Gewebe einer Wand eines Organs einzudringen, und an seinem proximalen Ende mit einem Kupplungsorgan (50) versehen ist, das imstande ist, mit einem angepassten Kupplungsorgan (32) des Werkzeugs zusammenzuwirken;
das Werkzeug einen Katheter (26) umfasst, der an seinem distalen Ende mit dem angepassten Kupplungsorgan (32) versehen ist;
wobei das Kupplungsorgan (50) der Kapsel und das angepasste Kupplungsorgan (32) des Werkzeugs komplementäre jeweilige Formen (54, 56, 58, 60; 36, 38, 40) aufweisen, die imstande sind, die Übertragung eines Moments für den Rotationsantrieb der Kapsel durch den Katheter zu gestatten;
wobei die Anordnung ferner ein Verbindungsmittel (46, 48, 62) umfasst, das imstande ist, selektiv in axialer Richtung eine Kraft zur gegenseitigen Annäherung und zur Anlage des Kupplungsorgans (50) der Kapsel gegen das angepasste Kupplungsorgan (32) des Werkzeugs zu erzeugen, die in der Lage ist, die Kapsel (10) mit dem Katheter (26) in Rotation und Translation fest zu verbinden und dann die Kapsel von dem Katheter durch eine Zurücknahme der Kraft zu entkoppeln,
wobei die Anordnung **dadurch gekennzeichnet ist, dass**:
die Form des angepassten Kupplungsorgan (32) des Werkzeugs auf der distalen Seite, im Querschnitt durch eine axiale Ebene, eine Ausnehmung (36) umfasst, die zu der Rückseite der Kapsel hin weist, mit einer V-Form (36), die zwei geneigte Flächen umfasst, und
die Form des Kupplungsorgan (50) der Kapsel an ihrem proximalen Ende eine konvexe Fläche (60) umfasst, die imstande ist, reibend und gleitend gegen die divergierenden geneigten Flächen (38) der V-Form (36) in Anlage zu kommen.

2. Anordnung nach Anspruch 1, wobei die V-Form (36) eine Diederform mit zwei divergierenden geneigten Flächen (38) ist, die in jeweiligen Ebenen verlaufen.

3. Anordnung nach Anspruch 2, wobei die Schnittgerade (d) des Dieders radial verläuft.

4. Anordnung nach Anspruch 2, wobei der Winkel des Dieders zwischen 10° und 80° beträgt, bevorzugt zwischen 30° und 60° beträgt.

5. Anordnung nach Anspruch 1, wobei das angepasste Kupplungsorgan (32) des Werkzeugs auf der distalen Seite eine Stirnfläche umfasst, die imstande ist, gegen das proximale Ende der Kapsel in Anlage zu kommen, und die zwei Stirnhalbflächen (66) umfasst, die radial jede jeweilige geneigte Fläche (38) der V-Form (36) fortsetzen.

6. Anordnung nach Anspruch 5, wobei die zwei Stirnhalbflächen (66) in jeweiligen geneigten Flächen in gegenüber einer radialen Ebene entgegengesetzten Richtungen verlaufen.

7. Anordnung nach Anspruch 6, wobei der Neigungswinkel der Ebenen der jeweiligen Stirnhalbflächen (66) gegenüber der radialen Ebene zwischen 5° und 15° beträgt.

8. Anordnung nach Anspruch 1, wobei das angepasste Kupplungsorgan (32) des Werkzeugs auf der proximalen Seite mit dem Katheter durch ein aufgeweitetes Anschlussteil verbunden ist, dessen Abmessung im radialen Querschnitt von dem Katheter zu dem angepassten Kupplungsorgan hin progressiv zunimmt.

9. Anordnung nach Anspruch 1, wobei die konvexe Fläche (60) des Kupplungsorgans der Kapsel eine zylindrische Fläche ist, deren Achse (d) radial ausgerichtet ist.

10. Anordnung nach Anspruch 1, wobei das Material des Kupplungsorgans (50) der Kapsel ein metallisches Material ist und das Material des angepassten Kupplungsorgans (36) des Werkzeugs ein polymerer Kunststoff ist.

11. Anordnung nach Anspruch 1, wobei:
das Material des Kupplungsorgans der Kapsel,
das Material des angepassten Kupplungsorgans des Werkzeugs,
die Form des Kupplungsorgans der Kapsel und
die Form des angepassten Kupplungsorgans des Werkzeugs gemeinsam so gewählt sind, dass sie, an der Kontaktschnittstelle zwischen der Form des Kupplungsorgans (50) der Kapsel und der Form des angepassten Kupplungsorgans (36) des Werkzeugs, eine Kontaktreibungskraft erzeugen, die ausreicht, um
die Kapsel (10) mit dem Katheter (26) zu koppeln und den Rotationsantrieb der Kapsel durch den Katheter solange zu gestatten, wie ein Reaktionsmoment, das durch das Verankerungsorgan der Kapsel ausgeübt wird, kleiner ist als ein vorher bestimmtes Schwellenmoment, und
die Kapsel (10) von dem Katheter (26) zu entkoppeln, sobald das Reaktionsmoment das vorher bestimmte Schwellenmoment übersteigt, und so die Übertragung des Rotationsantriebsmoment auf die Kapsel zu unterbrechen.

12. Anordnung nach Anspruch 11, wobei das vorher bestimmte Schwellenmoment kleiner als 5 N.cm ist.

13. Anordnung nach Anspruch 1, wobei:
das Verbindungsmittel einen Rückhaltedraht (46, 48) umfasst, der in einem Schlitz (44) des Katheters (26) aufgenommen ist und an dem distalen Ende (44) des Katheters ausmündet, und
der Rückhaltedraht (46, 48) an dem Kupplungsorgan (50) der Kapsel so befestigt ist, dass ein axialer Zug, der in proximaler Richtung auf den Rückhaltedraht ausgeübt wird, das Kupplungsorgan (50) der Kapsel zur Annäherung gegen das angepasste Kupplungsorgans (36) des Werkzeugs beaufschlagt.

14. Anordnung nach Anspruch 1, wobei das Werkzeug ferner eine rohrförmige Schutzhülle (30) umfasst, die von dem distalen Ende des Katheters (26) getragen wird und ein Innenvolumen definiert, das zur Aufnahme, mit einem Freiheitsgrad in axialer Verschiebung, imstande ist von: wenigstens dem proximalen Bereich (52, 54) des rohrförmigen Körpers (12) der Kapsel, der das Kupplungsorgan (50) der Kapsel enthält; dem angepassten Kupplungsorgan (36) des Werkzeugs; und dem Verbindungsmittel (46, 48, 62).

## Claims

1. A unit comprising an autonomous implantable capsule (10) and a tool (26, 30) for guiding and anchoring the capsule to an implantation site (18), wherein:
the capsule comprises a tubular body (12) provided at its distal end with an anchoring member (14) adapted to enter a tissue of an organ wall, and at its proximal end with a coupling member (50) adapted to cooperate with a conjugated coupling member (32) of the tool;
the tool comprises a catheter (26) provided at its distal end with said conjugated coupling member (32);
the coupling member (50) of the capsule and the conjugated coupling member (32) of the tool having complementary respective shapes (54, 56, 58, 60 ; 36, 38, 40) adapted to allow the transmission of a torque rotationally driving the capsule by the catheter;
the unit further comprising a junction means (46, 48, 62) adapted to selectively produce in axial direction an effort for mutually moving closer and urging the coupling member (50) of the capsule against the conjugated coupling member (32) of the tool, for making the capsule (10) rotationally and translationally integral with the catheter (26), then uncoupling the capsule from the catheter by relieving said effort,
said unit being **characterized in that**:
the shape of the conjugated coupling member (32) of the tool comprises on the distal side, in axial cross-section, a recess (36) turned towards the rear of the capsule, with a V-shape (36) comprising two diverging, inclined faces (38), and
the shape of the coupling member (50) of the capsule comprises at its proximal end a convex surface (60) adapted to frictionally and slidingly urge against the diverging, inclined faces (38) of the V-shape (36).

2. The unit of claim 1, wherein the V-shape (36) is a dihedral shape with two diverging, inclined faces (38) extending in respective planes.

3. The unit of claim 2, wherein the intersecting straight line (d) of the dihedron extends radially.

4. The unit of claim 2, wherein the angle of the dihedron is comprised between 10° and 80°, preferably comprised between 30° and 60°.

5. The unit of claim 1, wherein the conjugated coupling member (32) of the tool comprises on the distal side a front surface adapted to urge against the proximal end of the capsule and comprising two front half-faces (66) radially extending each respective diverging, inclined face (38) of the V-shape (36).

6. The unit of claim 5, wherein the two front half-faces (66) extend in respective planes inclined in opposite directions with respect to a radial plane.

7. The unit of claim 6, wherein the angle of inclination of the planes of the respective front half-faces (66) with respect to the radial plane is comprised between 5° and 15°.

8. The unit of claim 1, wherein the conjugated coupling member (32) of the tool is connected on the proximal side to the catheter by a flared connection portion which size in radial cross-section progressively increases from the catheter towards the conjugated coupling member.

9. The unit of claim 1, wherein the convex surface (60) of the coupling member of the capsule is a cylindrical surface which axis (d) is radially orientated.

10. The unit of claim 1, wherein the material of the coupling member (50) of the capsule is a metallic material, and the material of the conjugated coupling member (36) of the tool is a polymeric plastic.

11. The unit of claim 1, wherein:
the material of the coupling member of the capsule,
the material of the conjugated coupling member of the tool,
the shape of the coupling member of the capsule, and
the shape of the conjugated coupling member of the tool are jointly chosen for producing, at the interface of contact between the shape of the coupling member (50) of the capsule and the shape of the conjugated coupling member (36) of the tool, a frictional contact force sufficient to:
couple the capsule (10) to the catheter (26) and allow rotationally driving the capsule by the catheter as long as a reaction torque exerted by the capsule anchoring member is lower than a predetermined threshold torque; and
uncouple the capsule (10) from the catheter (26) as soon as the reaction torque exceeds the predetermined threshold torque, whereby interrupting the transmission of the rotational driving torque to the capsule.

12. The unit of claim 11, wherein the predetermined threshold torque is lower than 5 N.cm.

13. The unit of claim 1, wherein:
the junction means comprises a retainer wire (46, 48) housed in a lumen (44) of the catheter (26) and exiting (44) at the distal end of the catheter, and
the retainer wire (46, 48) is fastened to the coupling member (50) of the capsule, so that an axial traction exerted in proximal direction on the retainer wire pushes the coupling member (50) of the capsule closer against the conjugated coupling member (36) of the tool.

14. The unit of claim 1, wherein the tool further comprises a tubular protective sleeve (30) carried by the distal end of the catheter (26) and defining an inner volume capable of housing, with an axial sliding degree of freedom: at least the proximal region (52, 54) of the tubular body (12) of the capsule including the coupling member (50) of the capsule; the conjugated coupling member (36) of the tool; and the junction means (46, 48, 62).
